# EUROPÄISCHE PATENTANMELDUNG

(11) **EP 0 714 982 A2**
(43) Veröffentlichungstag der Anmeldung: **05.06.1996**
(21) Anmeldenummer: 95118050.4
(22) Anmeldetag: 16.11.1995
(51) Int. Cl.: C12N 15/62, C12N 9/64, C12N 9/72, C07K 14/815, A61K 38/49, A61K 38/55

(54) **Chimäre Proteine mit fibrinolytischen und thrombinhemmenden Eigenschaften**

(30) Priorität: 30.11.1994 DE 4442665
(71) Anmelder: Grünenthal GmbH, D-52078 Aachen (DE)
(72) Erfinder: Wnendt, Stephan, Dr., D-52076 Aachen (DE); Steffens, Gerd Josef, Prof. Dr., D-52072 Aachen (DE); Janocha, Elke, D-52441 Linnich (DE); Heinzel-Wieland, Regina, Dr., D-64295 Darmstadt (DE)

(57) **Zusammenfassung**

Es werden chimäre Proteine mit fibrinolytischen und thrombinhemmenden Eigenschaften beschrieben, die am C-terminalen Ende der Plasminogen aktivierenden Aminosäuresequenz mit einer Thrombin hemmenden Aminosäuresequenz verknüpft sind.

SEQUENZPROTOKOLL

(1) ALLGEMEINE ANGABEN:

(i) ANMELDER:
(A) NAME: Gruenenthal GmbH
(B) STRASSE: Zieglerstrasse 6
(C) ORT: Aachen
(E) LAND: Deutschland
(F) POSTLEITZAHL: 52078
(G) TELEFON: 0241/5692599
(H) TELEFAX: 0241/5692544

(ii) BEZEICHNUNG DER ERFINDUNG: Chimaere Proteine mit fibrinolytischen
und thrombinhemmenden Eigenschaften

(iii) ANZAHL DER SEQUENZEN: 25

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 16 Aminosäuren
(B) ART: Aminosäure
(C) STRANGFORM:
(D) TOPOLOGIE: linear

(ii) ART DES MOLEKÜLS: Peptid

(v) ART DES FRAGMENTS: C-Terminus

(ix) MERKMAL:
(A) NAME/SCHLÜSSEL: Modified-site
(B) LAGE:2..6
(D) SONSTIGE ANGABEN:/product= "Xaa"
/label= Xaa
/note= "Pos 2: Xaa = Pro, Leu;
Pos 3: Xaa = Gly, Val, Pro
Pos 4: Xaa = Lys, Val, Arg, Gly, Glu
Pos 5: Xaa = Ala, Val, Gly, Leu, Ile
Pos 6: Xaa = Gly, Phe, Trp, Tyr, Val."

(ix) MERKMAL:
(A) NAME/SCHLÜSSEL: Modified-site
(B) LAGE:9..16
(D) SONSTIGE ANGABEN:/product= "Xaa"
/label= Xaa
/note= "Pos 10: Xaa = Phe, Tyr, Trp
Pos 11: Xaa = Leu, Ala, Gly, Ile, Ser, Met
Pos 12: Xaa = Leu, Ala, Gly, Ile, Ser, Met;
Pos 13: Xaa = Arg, Lys, His
Pos 16: Xaa = SEQ ID NO: 2, SEQ ID NO: 3,
SEQ ID NO: 4, SEQ ID NO: 5"

(xi) SEQUENZBESCHREIBUNG: SEQ ID NO: 1:

Ser Xaa Xaa Xaa Xaa Xaa Pro Arg Pro Xaa Xaa Xaa Xaa Asn Pro Xaa
1               5                   10                  15

(2) ANGABEN ZU SEQ ID NO: 2:

(i) SEQUENZKENNZEICHEN:
(A) LÄNGE: 12 Aminosäuren
(B) ART: Aminosäure
(C) STRANGFORM:
(D) TOPOLOGIE: linear

(ii) ART DES MOLEKÜLS: Peptid

(v) ART DES FRAGMENTS: C-Terminus

(ix) MERKMAL:
(A) NAME/SCHLÜSSEL: Modified-site
(B) LAGE:2..4
(D) SONSTIGE ANGABEN:/product= "Xaa"
/label= Xaa
/note= "Pos 3: Xaa = Phe, Tyr"

(xi) SEQUENZBESCHREIBUNG: SEQ ID NO: 2:

Gly Asp Xaa Glu Glu Ile Pro Glu Glu Tyr Leu Gln
1               5                   10

(2) ANGABEN ZU SEQ ID NO: 3:

(i) SEQUENZKENNZEICHEN:
(A) LÄNGE: 12 Aminosäuren
(B) ART: Aminosäure
(C) STRANGFORM:
(D) TOPOLOGIE: linear

(ii) ART DES MOLEKÜLS: Peptid

(v) ART DES FRAGMENTS: C-Terminus

(xi) SEQUENZBESCHREIBUNG: SEQ ID NO: 3:

Asn Asp Lys Tyr Glu Pro Phe Glu Glu Tyr Leu Gln
1               5                   10

(2) ANGABEN ZU SEQ ID NO: 4:

(i) SEQUENZKENNZEICHEN:
(A) LÄNGE: 13 Aminosäuren
(B) ART: Aminosäure
(C) STRANGFORM:
(D) TOPOLOGIE: linear

(ii) ART DES MOLEKÜLS: Peptid

(v) ART DES FRAGMENTS: C-Terminus

(xi) SEQUENZBESCHREIBUNG: SEQ ID NO: 4:

Ser Asp Phe Glu Glu Phe Ser Leu Asp Asp Ile Glu Gln
1               5                   10

(2) ANGABEN ZU SEQ ID NO: 5:

(i) SEQUENZKENNZEICHEN:
(A) LÄNGE: 13 Aminosäuren
(B) ART: Aminosäure
(C) STRANGFORM:
(D) TOPOLOGIE: linear

(ii) ART DES MOLEKÜLS: Peptid

(v) ART DES FRAGMENTS: C-Terminus

(xi) SEQUENZBESCHREIBUNG: SEQ ID NO: 5:

Ser Glu Phe Glu Glu Phe Glu Ile Asp Glu Glu Glu Lys
1               5                   10

(2) ANGABEN ZU SEQ ID NO: 6:

(i) SEQUENZKENNZEICHEN:
(A) LÄNGE: 63 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O105"

(iii) HYPOTHETISCH: NEIN

(iv) ANTISENSE: NEIN

(xi) SEQUENZBESCHREIBUNG: SEQ ID NO: 6:

TATGAGCAAA ACTTGCTACG AAGGTAACGG TCACTTCTAC CGTGGTAAGG CTTCTACCGA 60

CAC 63

(2) ANGABEN ZU SEQ ID NO: 7:

(i) SEQUENZKENNZEICHEN:
(A) LÄNGE: 65 Basenpaare
(B) ART: Nucleotid
(C) STRANGFORM: Einzelstrang

(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O106"

(iii) HYPOTHETISCH: NEIN

(iv) ANTISENSE: NEIN

(xi) SEQUENZBESCHREIBUNG: SEQ ID NO: 7:

CATGGTGTCG GTAGAAGCCT TACCACGGTA GAAGTGACCG TTACCTTCGT AGCAAGTTTT 60

GCTCA 65

(2) ANGABEN ZU SEQ ID NO: 8:

(i) SEQUENZKENNZEICHEN:
(A) LÄNGE: 83 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O220"

(iii) HYPOTHETISCH: NEIN

(iv) ANTISENSE: NEIN

(xi) SEQUENZBESCHREIBUNG: SEQ ID NO: 8:

CGGTTAAGGC TTTCCCGAGG CCTGGTGGTG GTGGTAACGG TGACTTCGAA GAAATCCCGG 60

AAGAGTACCT GTGATAGGAT CAA 83

(2) ANGABEN ZU SEQ ID NO: 9:

(i) SEQUENZKENNZEICHEN:
(A) LÄNGE: 91 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O221"

(iii) HYPOTHETISCH: NEIN

(iv) ANTISENSE: NEIN

(xi) SEQUENZBESCHREIBUNG: SEQ ID NO: 9:

CTAGTTGATC CTATCACAGG TACTCTTCCG GGATTTCTTC GAAGTCACCG TTACCACCAC 60

CACCAGGCCT CGGGAAAGCC TTAACCGGGC T 91

(2) ANGABEN ZU SEQ ID NO: 10:

(i) SEQUENZKENNZEICHEN:
(A) LÄNGE: 58 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O265"

(iii) HYPOTHETISCH: NEIN

(iv) ANTISENSE: NEIN

(xi) SEQUENZBESCHREIBUNG: SEQ ID NO: 10:

CACCCGGCGG AGACGGCGGG CTCAGAGCCA GACCGTTTTC TTCTTTGGTG TGAGAACG 58

(2) ANGABEN ZU SEQ ID NO: 11:

(i) SEQUENZKENNZEICHEN:
(A) LÄNGE: 58 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O281"

(iii) HYPOTHETISCH: NEIN

(iv) ANTISENSE: NEIN

(xi) SEQUENZBESCHREIBUNG: SEQ ID NO: 11:

CGTCCGGGTG GTGGTGGTAA CGGTGACTTC GAAGAAATCC CGGAAGAATA CCTGTAAG 58

(2) ANGABEN ZU SEQ ID NO: 12:

(i) SEQUENZKENNZEICHEN:
(A) LÄNGE: 70 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "Synthetische DNA;
Nukleotidsequenz fuer Oligo O282"

(iii) HYPOTHETISCH: NEIN

(iv) ANTISENSE: NEIN

(xi) SEQUENZBESCHREIBUNG: SEQ ID NO: 12:

GATCCGTTCT CACACCAAAG AAGAAAACGG TCTGGCTCTG AGCCCGCCGT CTCCGCCGGG 60

TGGTTTCCCG 70

(2) ANGABEN ZU SEQ ID NO: 13:

(i) SEQUENZKENNZEICHEN:
(A) LÄNGE: 70 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O283"

(iii) HYPOTHETISCH: NEIN

(iv) ANTISENSE: NEIN

(xi) SEQUENZBESCHREIBUNG: SEQ ID NO: 13:

CTAGCTTACA GGTATTCTTC CGGGATTTCT TCGAAGTCAC CGTTACCACC ACCACCCGGA 60

CGCGGGAAAC 70

(2) ANGABEN ZU SEQ ID NO: 14:

(i) SEQUENZKENNZEICHEN:
(A) LÄNGE: 30 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O329"

(iii) HYPOTHETISCH: NEIN

(iv) ANTISENSE: NEIN

(xi) SEQUENZBESCHREIBUNG: SEQ ID NO: 14:

AAGAAATCCC GGAAGAATAC CTGCAATAAG 30

(2) ANGABEN ZU SEQ ID NO: 15:

(i) SEQUENZKENNZEICHEN:
(A) LÄNGE: 55 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O330"

(iii) HYPOTHETISCH: NEIN

(iv) ANTISENSE: NEIN

(xi) SEQUENZBESCHREIBUNG: SEQ ID NO: 15:

CGGTTAAGGC CTTGGGGACC GCGGCCGCTG GGTGGTGGTG GTAACGGTGA CTTCG 55

(2) ANGABEN ZU SEQ ID NO: 16:

(i) SEQUENZKENNZEICHEN:
(A) LÄNGE: 42 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O331"

(iii) HYPOTHETISCH: NEIN

(iv) ANTISENSE: NEIN

(xi) SEQUENZBESCHREIBUNG: SEQ ID NO: 16:

ACCACCACCC AGCGGCCGCG GTCCCCAAGC CTTAACCGGG CT 42

(2) ANGABEN ZU SEQ ID NO: 17:

(i) SEQUENZKENNZEICHEN:
(A) LÄNGE: 50 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O332"

(iii) HYPOTHETISCH: NEIN

(iv) ANTISENSE: NEIN

(xi) SEQUENZBESCHREIBUNG: SEQ ID NO: 17:

CTAGCTTATT GCAGGTATTC TTCCGGGATT TCTTCGAAGT CACCGTTACC 50

(2) ANGABEN ZU SEQ ID NO: 18:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O347"

(iii) HYPOTHETISCH: NEIN

(iv) ANTISENSE: NEIN

(xi) SEQUENZBESCHREIBUNG: SEQ ID NO: 18:

CGGTTGTTGC TTTCCCGC 18

(2) ANGABEN ZU SEQ ID NO: 19:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo O348"

(iii) HYPOTHETISCH: NEIN

(iv) ANTISENSE: NEIN

(xi) SEQUENZBESCHREIBUNG: SEQ ID NO: 19:

GGCCGCGGGA AAGCAACAAC CGGGCT 26

(2) ANGABEN ZU SEQ ID NO: 20:

(i) SEQUENZKENNZEICHEN:
(A) LÄNGE: 63 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo 0545"

(iii) HYPOTHETISCH: NEIN

(iv) ANTISENSE: NEIN

(xi) SEQUENZBESCHREIBUNG: SEQ ID NO: 20:

CTAGCTTATT GCAGGTATTC TTCGAACGGT TCGTATTTGT CGTTAGGGTT ACGCAGCAGG 60

AAA 63

(2) ANGABEN ZU SEQ ID NO: 21:

(i) SEQUENZKENNZEICHEN:
(A) LÄNGE: 63 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo 0546"

(iii) HYPOTHETISCH: NEIN

(iv) ANTISENSE: NEIN

(xi) SEQUENZBESCHREIBUNG: SEQ ID NO: 21:

GGCCTTTCCT GCTGCGTAAC CCTAACGACA AATACGAACC GTTCGAAGAA TACCTGCAAT 60

AAC 63

(2) ANGABEN ZU SEQ ID NO: 22:

(i) SEQUENZKENNZEICHEN:
(A) LÄNGE: 63 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo 0615"

(iii) HYPOTHETISCH: NEIN

(iv) ANTISENSE: NEIN

(xi) SEQUENZBESCHREIBUNG: SEQ ID NO: 22:

CTAGCTTATT GCAGGTATTC TTCCGGGATT TCTTCGAAGT CACCAGGGTT ACGCAGCAGG 60

AAA 63

(2) ANGABEN ZU SEQ ID NO: 23:

(i) SEQUENZKENNZEICHEN:
(A) LÄNGE: 63 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Synthetische DNA;
Nukleotidsequenz fuer Oligo 0618"

(iii) HYPOTHETISCH: NEIN

(iv) ANTISENSE: NEIN

(xi) SEQUENZBESCHREIBUNG: SEQ ID NO: 23:

GGCCTTTCCT GCTGCGTAAC CCTGGTGACT TCGAAGAAAT CCCGGAAGAA TACCTGCAAT 60

AAG 63

(2) ANGABEN ZU SEQ ID NO: 24:

(i) SEQUENZKENNZEICHEN:
(A) LÄNGE: 393 Aminosäuren
(B) ART: Aminosäure
(C) STRANGFORM:
(D) TOPOLOGIE: linear

(ii) ART DES MOLEKÜLS: Protein

(xi) SEQUENZBESCHREIBUNG: SEQ ID NO: 24:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Val Ala Phe Pro Arg Pro Phe Leu Leu Arg Asn Pro Gly Asp Phe
    370                 375                 380

Glu Glu Ile Pro Glu Glu Tyr Leu Gln
385                 390

(2) ANGABEN ZU SEQ ID NO: 25:

    (i) SEQUENZKENNZEICHEN:
        (A) LÄNGE: 393 Aminosäuren

(B) ART: Aminosäure
(C) STRANGFORM:
(D) TOPOLOGIE: linear

(ii) ART DES MOLEKÜLS: Protein

(xi) SEQUENZBESCHREIBUNG: SEQ ID NO: 25:

Met Ser Lys Thr Cys Tyr Glu Gly Asn Gly His Phe Tyr Arg Gly Lys
1               5                   10                  15

Ala Ser Thr Asp Thr Met Gly Arg Pro Cys Leu Pro Trp Asn Ser Ala
            20                  25                  30

Thr Val Leu Gln Gln Thr Tyr His Ala His Arg Ser Asp Ala Leu Gln
        35                  40                  45

Leu Gly Leu Gly Lys His Asn Tyr Cys Arg Asn Pro Asp Asn Arg Arg
    50                  55                  60

Arg Pro Trp Cys Tyr Val Gln Val Gly Leu Lys Pro Leu Val Gln Glu
65                  70                  75                  80

Cys Met Val His Asp Cys Ala Asp Gly Lys Lys Pro Ser Ser Pro Pro
                85                  90                  95

Glu Glu Leu Lys Phe Gln Cys Gly Gln Lys Thr Leu Arg Pro Arg Phe
            100                 105                 110

Lys Ile Ile Gly Gly Glu Phe Thr Thr Ile Glu Asn Gln Pro Trp Phe
        115                 120                 125

Ala Ala Ile Tyr Arg Arg His Arg Gly Gly Ser Val Thr Tyr Val Cys
    130                 135                 140

Gly Gly Ser Leu Ile Ser Pro Cys Trp Val Ile Ser Ala Thr His Cys
145                 150                 155                 160

Phe Ile Asp Tyr Pro Lys Lys Glu Asp Tyr Ile Val Tyr Leu Gly Arg
                165                 170                 175

Ser Arg Leu Asn Ser Asn Thr Gln Gly Glu Met Lys Phe Glu Val Glu
            180                 185                 190

Asn Leu Ile Leu His Lys Asp Tyr Ser Ala Asp Thr Leu Ala His His
        195                 200                 205

Asn Asp Ile Ala Leu Leu Lys Ile Arg Ser Lys Glu Gly Arg Cys Ala
    210                 215                 220

Gln Pro Ser Arg Thr Ile Gln Thr Ile Cys Leu Pro Ser Met Tyr Asn
225                 230                 235                 240

Asp Pro Gln Phe Gly Thr Ser Cys Glu Ile Thr Gly Phe Gly Lys Glu
                245                 250                 255

Asn Ser Thr Asp Tyr Leu Tyr Pro Glu Gln Leu Lys Met Thr Val Val
            260                 265                 270

Lys Leu Ile Ser His Arg Glu Cys Gln Gln Pro His Tyr Tyr Gly Ser
        275                 280                 285

Glu Val Thr Thr Lys Met Leu Cys Ala Ala Asp Pro Gln Trp Lys Thr
    290                 295                 300

Asp Ser Cys Gln Gly Asp Ser Gly Gly Pro Leu Val Cys Ser Leu Gln
305                 310                 315                 320

Gly Arg Met Thr Leu Thr Gly Ile Val Ser Trp Gly Arg Gly Cys Ala
                325                 330                 335

Leu Lys Asp Lys Pro Gly Val Tyr Thr Arg Val Ser His Phe Leu Pro
            340                 345                 350

Trp Ile Arg Ser His Thr Lys Glu Glu Asn Gly Leu Ala Leu Ser Pro
        355                 360                 365

Val Val Ala Phe Pro Arg Pro Phe Leu Leu Arg Asn Pro Asn Asp Lys
    370                 375                 380

Tyr Glu Pro Phe Glu Glu Tyr Leu Gln
385                 390

## Beschreibung

Die Erfindung betrifft chimäre Proteine mit fibrinolytischen und thrombinhemmenden Eigenschaften, die am C-terminalen Ende der Plasminogen aktivierenden Aminosäuresequenz mit einer Thrombin hemmenden Aminosäuresequenz verknüpft sind, Plasmide zur Herstellung dieser Polypeptide sowie Thrombolytika, die als Wirkstoff ein derartiges Polypeptid enthalten.

In allen Industrieländern stellen Herz-Kreislauferkrankungen zur Zeit die häufigste Todesursache dar. Besondere Bedeutung kommt dabei den akuten thrombotischen Verschlüssen zu, deren Entstehung im Falle des Herzinfarkts innerhalb kürzester Zeit zu einer lebensbedrohlichen Unterversorgung des Herzmuskels führt. Ähnliches gilt für den Hirninfarkt, wobei hier die intracerebralen Verschlüsse mit massiven ischämischen Schädigungen der betroffenen Hirnareale einhergehen. Im Gegensatz zum Herzinfarkt, der mit hohen Mortalitätsraten verbunden ist, führt die Unterversorgung beim Hirninfarkt in der Regel nicht zu akuten lebensbedrohlichen Situationen sondern durch Ausfall bestimmter Gehirnfunktionen zu einer starken Beeinträchtigung der täglichen Lebensweise und damit teilweise zu einem drastischen Verlust der Lebensqualität für die Betroffenen. Für beide Infarktformen gilt generell, daß die von den betroffenen Arterien versorgten Bezirke innerhalb weniger Stunden - ohne eine Therapie- irreversibel geschädigt werden. Weitere thrombotische Verschlußerkrankungen, die einer Behandlung bedürfen, sind die Lungenembolie, die Venenthrombose und periphere arterielle Verschlußererkrankungen.

Der durch einen Thrombus hervorgerufene Verschluß eines Blutgefäßes bildet sich meist an einer arteriosklerotischen Läsion aus Fibrin, Thrombozyten und Erythrozyten unter Einwirkung verschiedener Enzyme des Blutgerinnungssystems. Innerhalb der Enzymkaskade des Gerinnungssystems spielt Thrombin eine prominente Rolle. Thrombin kann alle wichtigen Enzyme des Gerinnungsystems aktivieren, die Aggregation von Thrombozyten induzieren und zur Bildung eines Fibringespinstes durch Umsetzung von Fibrinogen zu Fibrin führen (Furie und Furie in New Engl. J. Med. 326, 800 (1992)).

Die Bildung von Thromben wird durch physiologische Antikoagulantien, beispielsweise Antithrombin III, aktiviertes Protein C und Tissue Factor Pathway Inhibitor, begrenzt. Einmal gebildete Thromben können durch Einwirkung von körpereigenem Plasmin wieder aufgelöst werden. Plasmin entsteht aus einem inaktiven Proenzym, dem Plasminogen, das durch Plasminogenaktivatoren proteolytisch aktiviert wird. Die durch Plasmin hervorgerufene Thrombolyse wird therapeutisch genutzt, indem Patienten mit thrombotischen Erkrankungen, insbesondere Patienten mit aktutem Herzinfarkt, mit Plasminogenaktivatoren behandelt werden. Ziel der therapeutischen Intervention ist das betroffene Infarktareal zu verkleinern und die Mortalitätsrate zu senken. Zur Zeit stehen für diese Therapie Streptokinase, APSAC (Anisolated Plasminogen Streptokinase Activator Complex), zweikettige Urokinase (UK), rekombinante, einkettige Urokinase (rekombinante Prourokinase) und Gewebeplasminogenaktivator (t-PA) zur Verfügung (Collen und Lijnen in Blood 78, 3114 (1991)). Aus den bisher vorliegenen Erfahrungen der Lysetherapie geht eindeutig hervor, daß die Wiedereröffnung der verschlossenen Koronargefäße innerhalb weniger Stunden, d.h. 1 bis 4 Stunden nach dem Eintritt des Infarkts die besten funktionellen Ergebnisse liefert. Um das Ziel einer optimalen Reperfusion zu erreichen, müßte bei der Mehrzahl der Fälle der Therapiebeginn eigentlich noch vor der stationären Aufnahme beginnen. Dies ist aber nur mit einem nebenwirkungsarmen und sicheren Fibrinolytikum möglich, auch im Hinblick auf die zu diesem Zeitpunkt noch unsichere Diagnosestellung. Alle Fibrinolytika der sogenannten ersten Generation, wie Streptokinase, APSAC und Urokinase erzeugen jedoch in der notwendigen Dosierung bei der Behandlung des akuten Infarkts eine generalisierte Plasminogenaktivierung, was mit einem hohen Blutungsrisiko einhergeht. Auch die Verwendung von Fibrinolytika der sogenannten zweiten Generation, t-PA und Prourokinase führt bei vielen Infarktpatienten zu einer systemischen Plasminogenaktivierung. Zur erfolgreichen Reperfusion und zur Vermeidung von Reokklusionen müssen sowohl t-PA als auch Prourokinase in hohen Dosen eingesetzt werden, die zu einer deutlichen Fibrinogenolyse, also einer systemischen Plasminogenaktivierung führen. Dies stimmt mit der Beobachtung überein, daß in den bisherigen Studien keine signifikanten Unterschiede in der Häufigkeit von Blutungskomplikationen zwischen den mit tPA oder Prourokinase und den mit Streptokinase behandelten Patienten nachgewiesen werden konnten.

Es werden daher verschiedene Ansätze verfolgt, um das pharmakologische Profil von Plasminogenaktivatoren zu verbessern. In der Entwicklung befinden sich Fledermaus-Plasminogenaktivatoren (Gardell et al. in J. Biol. Chem. 264, 17947 (1989); EP 383 417), Staphylokinase (Schlott et al. in Bio/Technology 12, 185 (1994); Collen und Van De Werf in Circulation 87, 1850 (1993)), der rekombinante Gewebeplasminogenaktivator BM 06.022 (Martin et al. in J. Cardiovasc. Pharm. 18, 111 (1991)) sowie die t-PA-Variante TNK-t-PA (Keyt et al. in Proc. Natl. Acad. Sci. 91, 3670 (1994)).

Streptokinase, ein Protein hämolytischer Streptokokken, aktiviert humanes Plasminogen, indem es einen Komplex mit Plasminogen bildet und dadurch das Plasminogen in eine aktive Konformation überführt. Dieser Komplex selbst setzt freies Plasminogen zu Plasmin um, welches dann wiederum das an Streptokinase gebundene Plasminogen spaltet. Ähnlich wirkt auch Staphylokinase, ein aus Staphylococcus aureus gewonnenes Protein, das jedoch im Vergleich zu Streptokinase eine höhere Fibrinspezifität besitzt. Eine Weiterentwicklung der Streptokinase ist APSAC, eine in-vitro hergestellte Verbindung aus Streptokinase und menschlichem Plasminogen. APSAC besitzt aufgrund einer chemischen Modifikation des aktiven Zentrums des Plasminogens eine gegenüber Streptokinase erhöhte biologische Halbwertszeit.

Urokinase ist ein menschliches Protein, das in zwei Formen als proteolytisch aktives Protein aus Urin gewonnen werden kann: der hochmolekularen Urokinase (HUK) und der niedermolekularen Urokinase (LUK) (Stump et al. in J. Biol. Chem. 261, 1267 (1986)). HUK und LUK sind aktive Formen der Urokinase, d. h. Zweikettenmoleküle. Die Urokinase wird als einkettige Urokinase (Prourokinase) in verschiedenen Geweben gebildet und kann als Proenzym in geringen Mengen im menschlichen Blut nachgewiesen werden (Wun et al.in J. Biol. Chem. 257, 3276 (1982)). Die aktivierte Form der Prourokinase hat als HUK ein Molekulargewicht von 54 Kilodalton und besteht aus 3 Domänen: der amino-terminalen Growth-Factor-Domäne, dem Kringel und der Serin-Protease-Domäne (Günzler et al.in Hoppe-Seyler's Z. Physiol. Chem. 363, 1155 (1982); Steffens et al.in Hoppe-Seyler's Z. Physiol. Chem. 363, 1043 (1982)). Obwohl Prourokinase und Plasminogen als Proenzyme vorliegen, ist die Prourokinase aufgrund einer intrinsischen Aktivität in der Lage, Plasminogen in aktives Plasmin umzuwandeln. Die volle Aktivität erhält dieser Plasminogenaktivator aber erst, nachdem das gebildete Plasmin seinerseits die Prourokinase zwischen ¹⁵⁸Lysin und ¹⁵⁹Isoleucin gespalten hat (Lijnen et al.in J. Biol. Chem. 261, 1253 (1986)). Die gentechnische Gewinnung von Urokinase in Escherichia coli wurde erstmals von Heyneker et al. beschrieben (Proceedings of the IVth International Symposium on Genetics of Industrial Microorganisms 1982). Unglycosilierte Prourokinase (Saruplase) wird unter Verwendung eines synthetischen Gens hergestellt (Brigelius-Flohé et al.in Appl. Microbiol. Biotech. 36, 640 (1992)).

t-PA ist ein im Blut und im Gewebe vorkommendes Protein mit einem Molekulargewicht von 72 Kilodalton. Dieser Plasminogenaktivator besteht aus 5 Domänen: der aminoterminalen Finger-Domäne, der Growth-Factor-Domäne, dem Kringel 1, dem Kringel 2 und der Serin-Protease-Domäne. Wie Prourokinase wird t-PA durch eine plasminkatalysierte Spaltung zwischen Kringel 2 und der Serin-Protease-Domäne, d. h. zwischen ²⁷⁵Arg und ²⁷⁶Ile, in die aktive zweikettige Form überführt. In vitro-Studien und tierexperimentelle Ergebnisse weisen darauf hin, daß t-PA an Fibrin bindet und in seiner enzymatischen Aktivität durch Fibrin stimuliert wird (Collen und Lijnen in Blood 78, 3114 (1991)). Durch die Fibrinspezifität von t-PA sollte vermieden werden, daß Plasmin im gesamten Blutsystem gebildet und in der Folge nicht nur Fibrin, sondern auch Fibrinogen abgebaut wird. Eine solche systemische Plasminogenaktivierung sowie ein starker Abbau des Fibrinogens sind unerwünscht, da dies das Blutungsrisiko erhöht. Allerdings zeigte sich in der therapeutischen Praxis, daß die aus den präklinischen Studien abgeleiteten Erwartungen hinsichtlich der Fibrinspezifität von t-PA nicht erfüllt werden. Aufgrund der kurzen biologischen Halbwertszeit von t-PA müssen, wie bereits erwähnt, hohe Dosen infundiert werden, die trotz der Fibrinspezifität zu einer systemischen Plasminogenaktivierung führen (Keyt et al. in Proc. Natl. Acad. Sci. 91, 3670 (1994)).

r-PA und TNK-t-PA sind t-PA-Varianten mit verbesserten Eigenschaften. Beim r-PA (BM 06.022) wurden die ersten drei t-PA-Domänen, d. h. die Fingerdomäne, die Growth-Factor-Domäne und der erste Kringel deletiert, so daß das verkürzte Molekül nur den zweiten Kringel und die Protease-Domäne enthält. r-PA wird gentechnisch in Escherischia coli hergestellt und ist nicht glykosyliert. Im Vergleich zu t-PA hat r-PA eine längere biologische Halbwertszeit und führt schneller zur Reperfusion. In Tierexperimenten zeigte sich, daß r-PA als Bolus appliziert gleich wirksam ist wie eine t-PA-Infusion (Martin et al. in J. Cardiovasc. Pharmacol. 18, 111 (1991)).

Die t-PA-Variante TNK-t-PA unterscheidet sich von natürlichem t-PA in drei Punkten: Austausch von ¹⁰³Threonin gegen Asparagin, wodurch eine neue Glykosylierungsstelle entstanden ist; Austausch von ¹¹⁷Asparagin gegen Glutamin, wodurch eine Glykosylierungsstelle entfernt ist und Austausch der Sequenz zwischen ²⁹⁶Lysin und ²⁹⁹Arginin gegen vier aufeinanderfolgende Alanin-Einheiten. Die Kombination dieser drei Mutationen ergibt ein Polypeptid mit höherer Fibrinspezifität und längerer biologischer Halbwertszeit verglichen mit natürlichem t-PA. Darüber hinaus ist TNK-t-PA wesentlich schlechter durch PAI-1 zu hemmen als natürliches t-PA (Keyt et al. in Proc. Natl. Acad. Sci. 91, 3670 (1994)). Tierexperimentelle Ergebnisse, die mit einem Vorläufer von TNK-t-PA erzielt wurden, weisen darauf hin, daß sich TNK-t-PA zur Bolusapplikation eignet (Refino et al. in Thromb. Haemost. 70, 313 (1993)).

Der Fledermausplasminogenaktivator (bat-PA) kommt im Speichel der Fledermaus Desmodus rotundus vor. Dieser inzwischen auch gentechnisch hergestellte Plasminogenaktivator hat eine noch ausgeprägtere Fibrinspezifität als t-PA und zeigt im Tierversuch eine verbesserte Thrombolyse bei erhöhter biologischer Halbwertszeit und verminderter systemischer Plasminogenaktivierung (Gardell et al. in Circulation 84, 244 (1991)).

Bei der Behandlung thrombotischer Erkrankungen werden Plasminogenaktivatoren in der Regel zusammen mit einer antikoagulativen Substanz, beispielsweise Heparin, verabreicht. Dadurch wird eine bessere Thrombolyse erreicht als bei alleiniger Behandlung mit einem Plasminogenaktivator (Tebbe et al. in Z. Kardiol. 80, Suppl. 3, 32 (1991)). Verschiedene Befunde aus der Klinik weisen darauf hin, daß parallel zur Auflösung der Thromben eine erhöhte Gerinnungsneigung auftritt (Szczeklik et al. in Arterioscl. Thromb. 12, 548 (1992); Goto et al. in Angiology 45, 273 (1994)). Es wird angenommen, daß hierfür Thrombinmoleküle verantwortlich sind, die im Thrombus eingeschlossen sind und bei der Auflösung des Gerinnsels wieder freigesetzt werden. Desweiteren gibt es Hinweise, daß auch Plasminogenaktivatoren selbst die Aktivierung von Prothrombin beschleunigen und damit der Thrombolyse entgegenwirken (Brommer und Meijer in Thromb. Haemostas. 70, 995 (1993)). Antikoagulative Substanzen wie Heparin, Hirugen, Hirudin, Argatroban, Protein C und rekombinantes Tick Anticoagulant Peptide (TAP) können die verstärkte Reokklusionsneigung während der Thrombolyse unterbinden und damit den Erfolg der Lysetherapie verbessern (Yao et al. in Am. J. Physiol. 262 (Heart Circ. Physiol. 31) H 347 - H 379 (1992); Schneider in Thromb. Res. 64, 667 (1991); Gruber et al. in Circulation 84, 2454 (1991); Martin et al. in J. Am. Coll. Cardiol. 22, 914 (1993); Vlasuk et al. in Circulation 84, Suppl. II-467 (1991).

Einer der stärksten Thrombininhibitoren ist das aus 65 Aminosäuren bestehende Hirudin aus dem Blutegel Hirudo medicinales. Es gibt verschiedene Hirudin-Isoformen, die sich in einigen Aminosäuren unterscheiden. Alle Hirudin-Isoformen blockieren sowohl die Anbindung des Thrombins an ein Substrat, beispielsweise Fibrinogen, als auch das aktive Zentrum des Thrombins (Rydel et al. in Science 249, 277 (1990); Bode und Huber in Molecular Aspects of Inflammation, Springer, Berlin, Heidelberg, 103 - 115 (1991); Stone und Hofsteenge in Prot. Engineering 2, 295 (1991); Dodt et al. in Biol. Chem. Hoppe-Seyler 366, 379 (1985). Darüber hinaus sind vom Hirudin abgeleitete kleinere Moleküle bekannt, die ebenfalls thrombininhibitorische Aktivität besitzen (Maraganore et al. in Biochemistry 29, 7095 (1990); Krstenansky et al. in J. Med. Chem. 30, 1688 (1987); Yue et al. in Prot. Engineering 5, 77 (1992)).

Die Verwendung von Hirudin in Kombination mit einem Plasminogenaktivator zur Behandlung thrombotischer Erkrankungen ist in den europäischen Patentanmeldungen EP 328 957 und EP 365 468 beschrieben. Die Verwendung von Hirudin-Derivaten in Kombination mit einem Thrombolytikum ist aus der internationalen Patentanmeldung WO 91/01142 bekannt.

Hirullin ist ein aus dem Blutegel Hirudo manillensis isoliertes Protein mit 61 Aminosäuren. Hirullin gleicht in seiner Wirkung und Inhibitorstärke Hirudin, weicht jedoch in der Aminosäuresequenz stark von Hirudin ab. Auch von Hirullin konnten kleinere Moleküle abgeleitet werden, die Thrombin sehr gut hemmen (Krstenansky et al. in Febs Lett. 269, 465 (1990)).

Desweiteren kann Thrombin auch durch ein Peptid gehemmt werden, das sich aus der amino-terminalen Sequenz des humanen Thrombinrezeptors ableitet (Vu et al. in Nature 253, 674 (1991)). Der Thrombinrezeptor enthält in der extrazellulären, amino-terminalen Region eine thrombinbindende Sequenz mit benachbarter Spaltstelle für Thrombin. Diese Sequenz kann Thrombin hemmen, sofern die Spaltstelle durch einen Austausch von ⁴²Serin in ⁴²Phenylalanin maskiert ist.

Phaneuf et al. beschreiben in Thromb. Haemost. 71, 481 (1994) einen Komplex, der aus einer zufälligen chemischen Verknüpfung von Streptokinase und Hirudin besteht. Die Fähigkeit, Plasminogen zu aktivieren, liegt jedoch bei diesem Streptokinase-Hirudin-Komplex um den Faktor 8 unter der unmodifizierten Streptokinase.

Die der Erfindung zugrundeliegende Aufgabe bestand in der Entwicklung von Wirkstoffen zur Behandlung thrombotisch bedingter Gefäßverschlüsse, die innerhalb sehr kurzer Zeit eine vollständige Thrombolyse bewirken und gleichzeitig den Wiederverschluß der Gefäße nach zunächst erfolgreicher Thrombolyse verhindern. Ferner soll mit diesen Wirkstoffen eine systemische Plasminogenaktivierung vermieden werden.

Es wurde nun gefunden, daß die an solche Wirkstoffe gestellten hohen Anforderungen von chimären Proteinen mit fibrinolytischen Eigenschaften erfüllt werden, die am C-terminalen Ende der Plasminogen aktivierenden Aminosäuresequenz eine Thrombin hemmende Aminosäuresequenz besitzen.

Gegenstand der Erfindung sind dementsprechend chimäre Proteine mit fibrinolytischen und thrombinhemmenden Eigenschaften, die am C-terminalen Ende der Plasminogen aktivierenden Aminosäuresequenz mit einer Aminosäuresequenz der Formel I (SEQ ID NO:1)
Ser-X₁-X₂-X₃-X₄-X₅-Pro-Arg-Pro-Y₁-Y₂-Y₃-Y₄-Asn-Pro-Z
verknüpft sind, in der X₁ Pro oder Leu, X₂ Gly, Val oder Pro, X₃ Lys, Val, Arg, Gly oder Glu, X₄ Ala, Val, Gly, Leu oder Ile, X₅ Gly, Phe, Trp, Tyr oder Val, Y₁ Phe, Tyr oder Trp, Y₂ Leu, Ala, Gly, Ile, Ser oder Met, Y₃ Leu, Ala, Gly, Ile, Ser oder Met, Y₄ Arg, Lys oder His und Z die Aminosäuresequenz der Formel II (SEQ ID NO:2)
Gly-Asp-Z₁-Glu-Glu-Ile-Pro-Glu-Glu-Tyr-Leu-Gln
mit Z₁ Phe oder Tyr
oder der Formel III (SEQ ID NO:3)
Asn-Asp-Lys-Tyr-Glu-Pro-Phe-Glu-Glu-Tyr-Leu-Gln
oder der Formel IV (SEQ ID NO:4)
Ser-Asp-Phe-Glu-Glu-Phe-Ser-Leu-Asp-Asp-Ile-Glu-Gln
oder der Formel V (SEQ ID NO:5)
Ser-Glu-Phe-Glu-Glu-Phe-Glu-Ile-Asp-Glu-Glu-Glu-Lys
bedeuten.

Die erfindungsgemäßen chimären Proteine binden über die Thrombin-hemmende Aminosäuresequenz der Formel I an Thrombin, wodurch am Gerinnsel hohe Konzentrationen an chimärem Protein erreicht werden. Da die beim akuten Herz- oder Hirninfarkt gebildeten Gerinnsel reich an Thrombin sind, bietet die Thrombusspezifität der erfindungsgemäßen Proteine die Möglichkeit, die thrombolytische Wirksamkeit und Selektivität der Plasminogenaktivatoren zu erhöhen. Hierdurch wird eine systemische Plasminogenaktivierung und Fibrinogenolyse vermieden und die Sicherheit der Wirkstoffe deutlich erhöht. Durch die Thrombusspezifität kann auch die Dosierung im Vergleich zu herkömmlichen Plasminogenaktivatoren verringert werden, was wiederum die Sicherheit des Präparates steigert. Gleichzeitig ist zu erwarten, daß die Dosierung der antikoagulativen Komedikamentation (z.B. mit Heparin) bei der Verwendung der erfindungsgemäßen Proteine reduziert werden kann. Darüber hinaus ist auch der Verzicht auf ein zusätzliches Antikoagulans möglich.

Bevorzugte chimäre Proteine enthalten als Plasminogen aktivierende Aminosäuresequenz die unveränderte Aminosäuresequenz der Prourokinase, mindestens eine durch Deletion, Substitution, Insertion und/oder Addition modifizierte Aminosäuresequenz der Prourokinase, die unveränderte Aminosäuresequenz der Urokinase, mindestens eine durch Deletion, Substitution, Insertion und/oder Addition modifizierte Aminosäuresequenz der Urokinase, die unveränderte Aminosäuresequenz des Gewebeplasminogenaktivators (t-PA), mindestens eine durch Deletion, Substitution, Insertion und/oder Addition modifizierte Aminosäuresequenz von t-PA, die unveränderte Aminosäuresequenz des Fledermaus-Plasminogenaktivators (bat-PA), mindestens eine durch Deletion, Substitution, Insertion und/oder Addition modifizierte Aminosäuresequenz von bat-PA, und/oder die Aminosäuresequenz von Streptokinase, Staphylokinase und/oder APSAC.

Die Plasminogen aktivierende Aminosäuresequenz in den erfindungsgemäßen Proteinen enthält insbesondere die unveränderte Aminosäuresequenz von Prourokinase, mindestens eine durch Deletion, Substitution, Insertion und/oder Addition modifizierte Aminosäuresequenz der Prourokinase, die unveränderte Aminosäuresequenz von t-PA und/oder mindestens eine durch Deletion, Substitution, Insertion und/oder Addition modifizierte Aminosäuresequenz von t-PA. Besonders bevorzugt werden Proteine, deren Plasminogen aktivierende Aminosäuresequenz aus der aus 411 Aminosäuren bestehenden Sequenz der Prourokinase, in der die Aminosäure in Position 407 Asn oder Gln ist, aus der Aminosäuresequenz ⁴⁷Ser bis ⁴¹¹Leu der Prourokinase, in der die Aminosäure in Position 407 Asn oder Gln ist, aus der Aminosäuresequenz ¹³⁸Ser bis ⁴¹¹Leu der Prourokinase, in der die Aminosäure in Position 407 Asn oder Gln ist, aus der unveränderten, aus 527 Aminosäuren bestehenden Sequenz von t-PA, aus der Aminosäuresequenz Ser-⁸⁹Arg bis ⁵²⁷Pro von t-PA und/oder aus der Aminosäuresequenz ¹⁷⁴Ser bis ⁵²⁷Pro von t-PA besteht.

In den chimären Proteinen ist die Plasminogen aktivierende Aminosäuresequenz am C-terminalen Ende vorzugsweise mit einer Thrombin hemmenden Aminosäuresequenz der Formel I verknüpft, in der X₁ Pro, X₂ Val, X₃ Lys oder Val, X₄ Ala und X₅ Phe bedeuten. In der Aminosäuresequenz der Formel I steht Y₁ vorzugsweise für Phe, Y₂ vorzugsweise für Leu, Y₃ vorzugsweise für Leu und Y₄ vorzugsweise für Arg. Die Variable Z in der Aminosäuresequenz der Formel I steht insbesondere für eine Aminosäuresequenz der Formeln II oder IV.

Im Vergleich zu bekannten Plasminogenaktivatoren, zu bekannten Mischungen aus einem Plasminogenaktivator und einem Thrombininhibitor sowie zu dem bekannten Streptokinase-Hirudin-Komplex zeichnen sich die erfindungsgemäßen Proteine durch eine stärkere fibrinolytische Wirkung, verbunden mit überraschend guten Thrombin inhibierenden Eigenschaften aus. Darüber hinaus wird von den erfindungsgemäßen Polypeptiden Plasmafibrinogen in deutlich geringeren Mengen verbraucht. Die hieraus resultierende signifikant höhere Fibrinspezifität, insbesondere auch im Vergleich zu den bekannten Mischungen eines Plasminogenaktivators und eines Thrombininhibitors, bewirkt, daß die Gerinnungsfähigkeit des Blutes nur wenig beeinflußt wird und die Gefahr von unkontrollierten Blutungen als mögliche Komplikation eines systemischen Fibrinogenabbaus minimiert ist. Die hohe Fibrinspezifität der erfindungsgemäßen Proteine ermöglicht somit Bolusapplikationen mit deutlich herabgesetzten Blutungsrisiko im Vergleich zu Bolusapplikationen bekannter Thrombolytika.

Weiterer Erfindungsgegenstand sind dementsprechend Thrombolytika, die als Wirkstoff ein erfindungsgemäßes Protein enthalten.

Zur Behandlung thrombotisch bedingter Gefäßverschlüsse, beispielsweise Herzinfarkt, Hirninfarkt, peripherer, akuter Arterienverschluß, Lungenembolie, instabile Angina Pectoris und tiefe Bein- und Beckenvenenthrombose, werden 0,1 bis 1 mg pro kg eines erfindungsgemäßen Polypeptides benötigt. Die erfindungsgemäßen Proteine können parenteral durch Bolusinjektion oder Infusion verabreicht werden.

Die erfindungsgemäßen Thrombolytika enthalten neben mindestens einem erfindungsgemäßen Polypeptid Hilfsstoffe, beispielsweise Trägermaterialien, Lösungsmittel, Verdünnungsmittel, Farbstoffe und Bindemittel. Die Auswahl dieser Hilfsstoffe sowie die einzusetzenden Mengen derselben hängt davon ab, wie das Arzneimittel appliziert werden soll und bereitet dem Fachmann keinerlei Probleme.

Die Herstellung der erfindungsgemäßen Proteine erfolgt mittels gentechnischer Verfahren. Dazu werden die entsprechenden Gene aus synthetischen Oligonukleotiden in geeignete Plasmide cloniert und unter Kontrolle des trp- oder tac-Promotors, insbesondere unter Kontrolle des trp-Promotors, in Escherischia coli exprimiert.

Erfindungsgegenstand sind dementsprechend auch Plasmide zur Verwendung bei der Herstellung chimärer Proteine, deren Operon einen regulierbaren Promotor, eine als Ribosomenbindestelle wirksame Shine-Dalgarno-Sequenz, ein Startcodon, ein synthetisches Strukturgen für ein erfindungsgemäßes Protein und stromabwärts vom Strukturgen ein oder zwei Terminatoren aufweist.

Die Expression der erfindungsgemäßen Plasmide wird in Escherischia coli-Stämmen, insbesondere in Escherischia coli-Stämmen der Gruppe K 12, beispielsweise E. coli K 12 JM 101 (ATCC 33876), E. coli K 12 JM 103 (ATCC 39403), E. coli K 12 JM 105 (DSM 4162) und E. coli K 12 DH 1 (ATCC 33849) durchgeführt. In der bakteriellen Zelle fallen die erfindungsgemäßen Polypeptide in hoher Ausbeute in Einschlußkörpern an, in denen das Protein in denaturierter Form vorliegt. Nach Isolierung der Einschlußkörper wird das denaturierte Protein proteinchemisch unter Einwirkung eines Redox-Systems in die gewünschte Tertiärstruktur zurückgefaltet.

### Beispiele

### 1. Darstellung, Isolierung und Reinigung erfindungsgemäßer Proteine

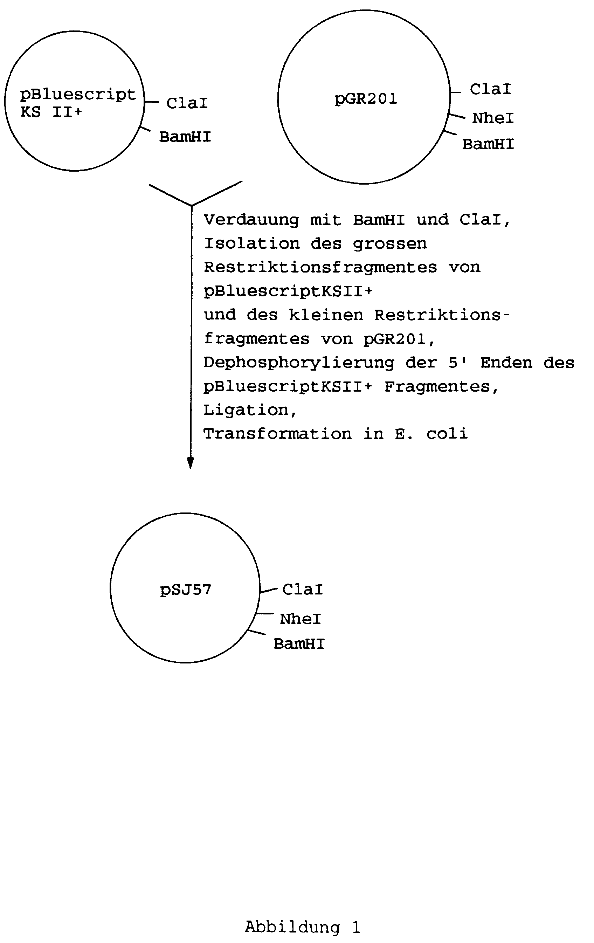

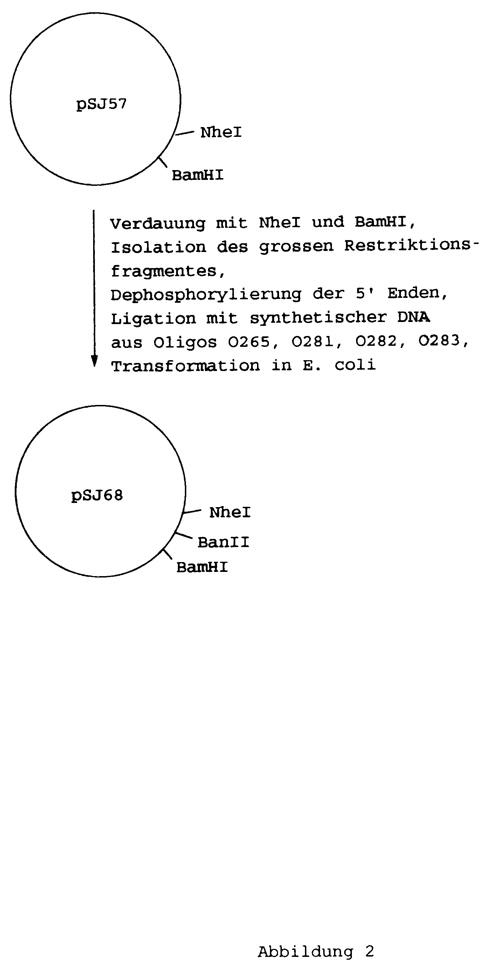

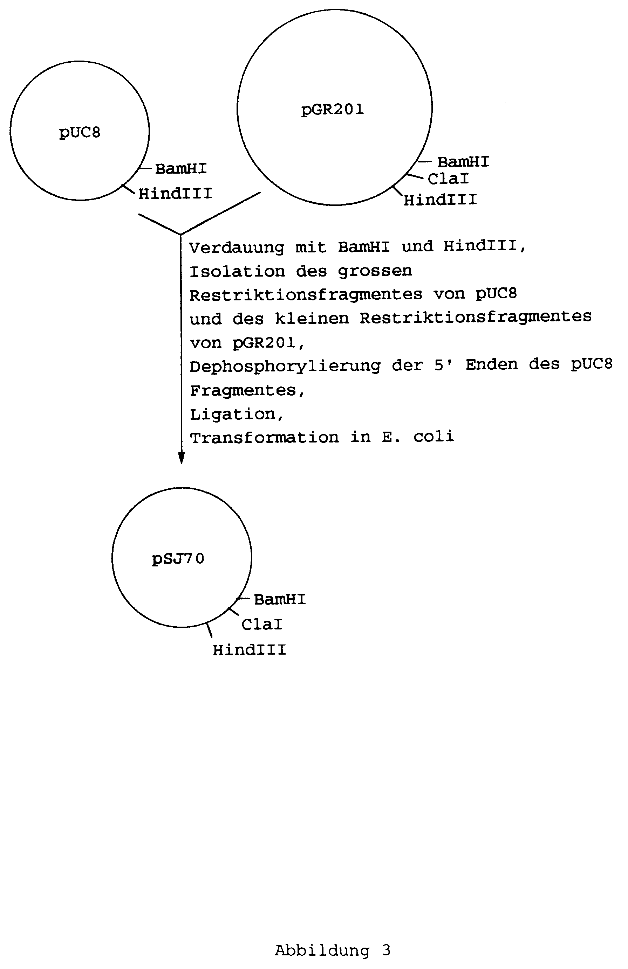

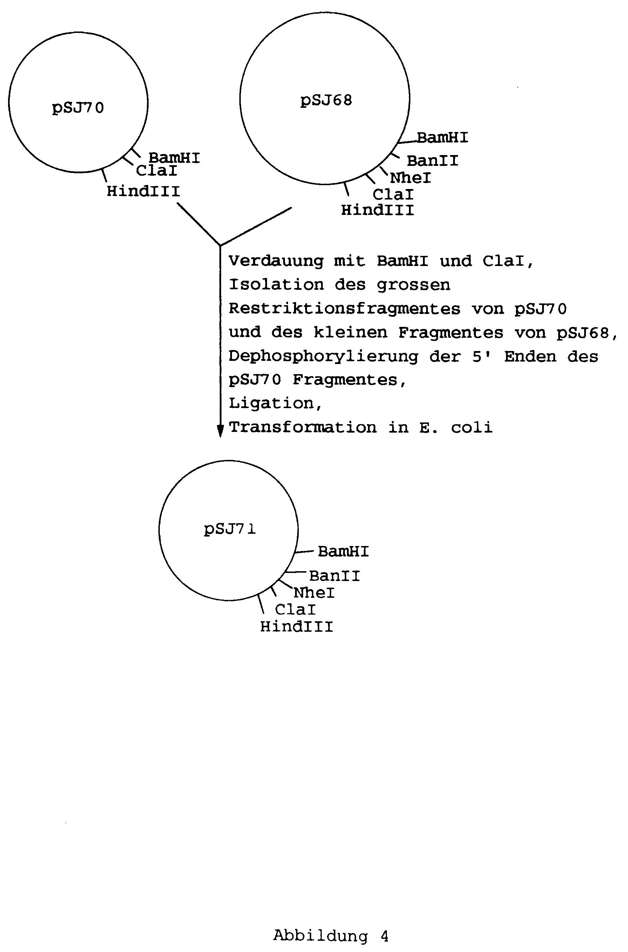

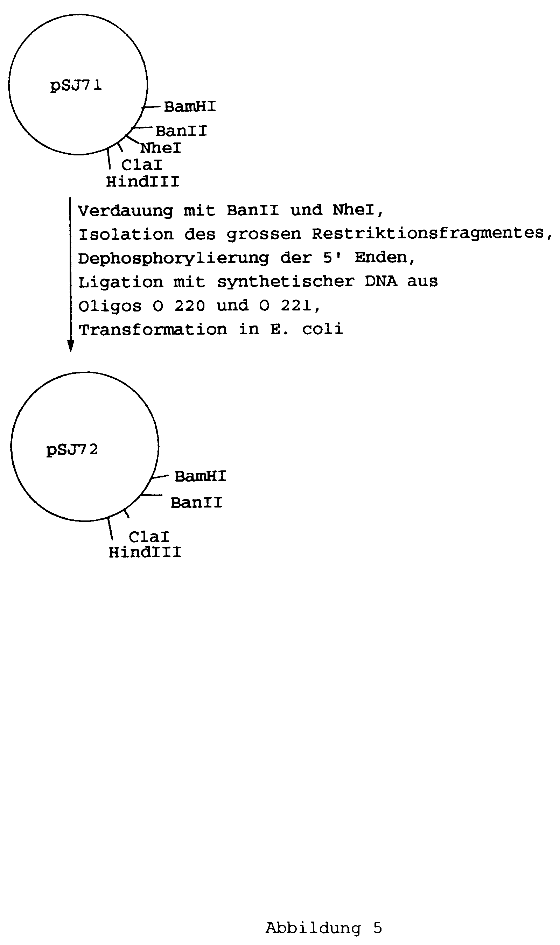

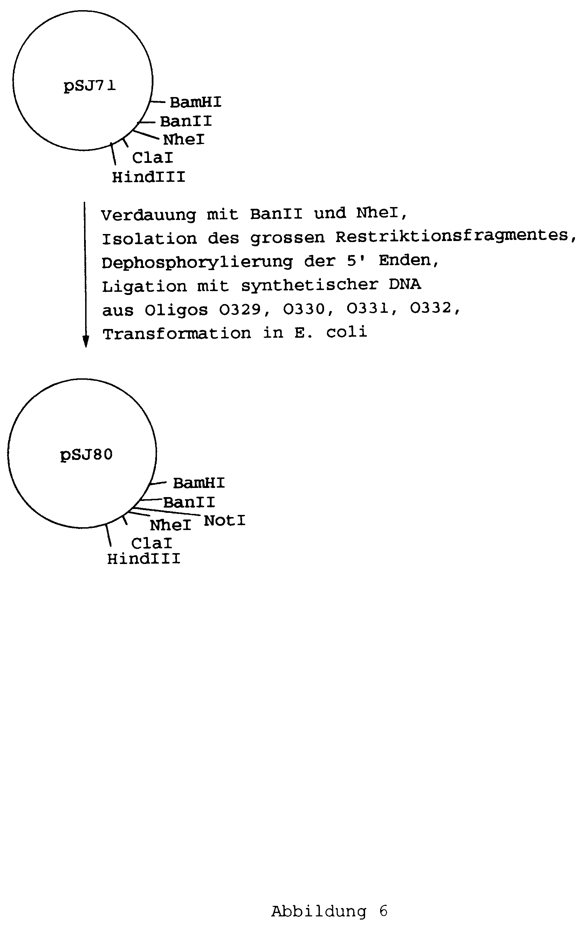

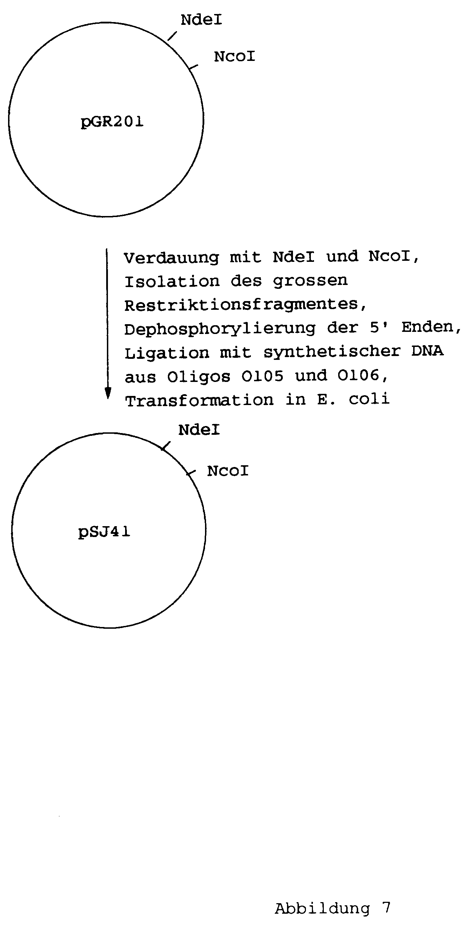

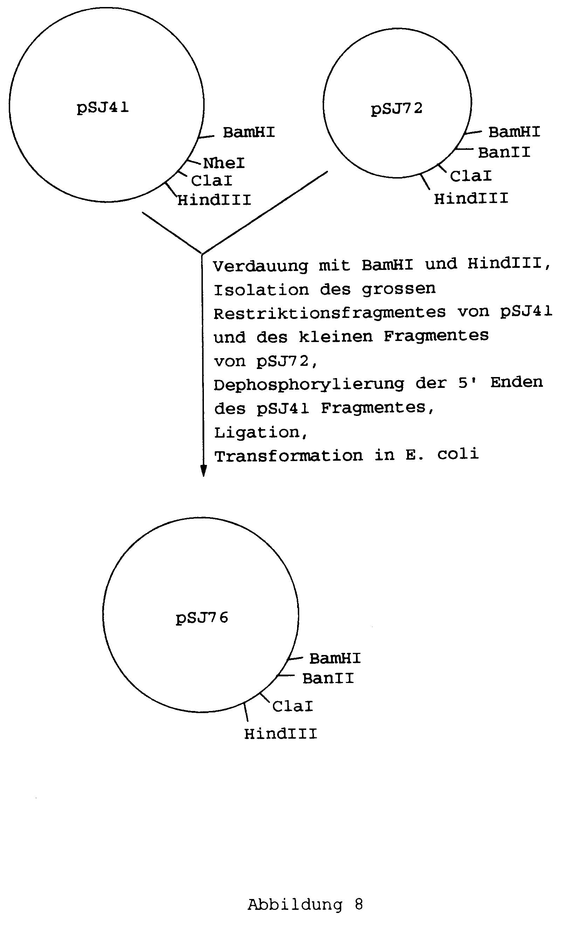

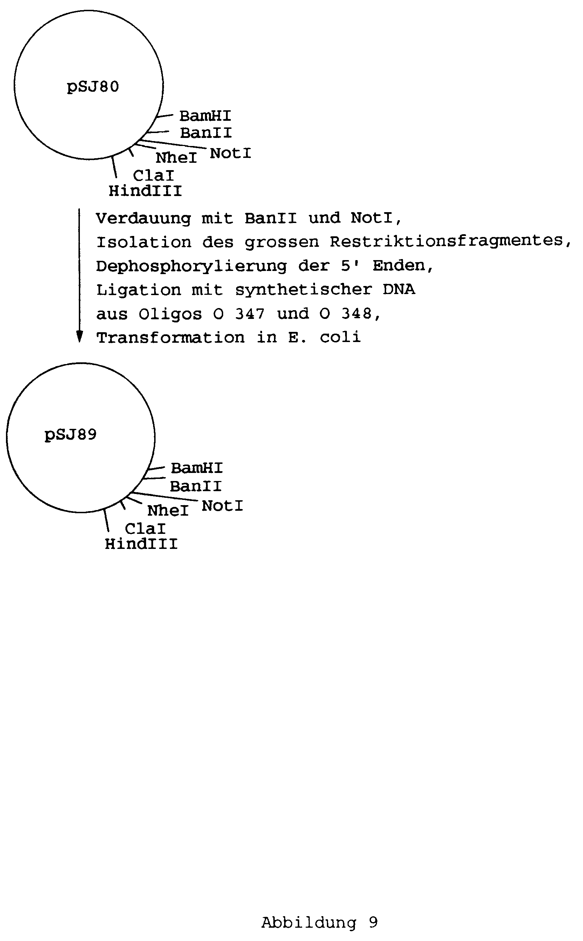

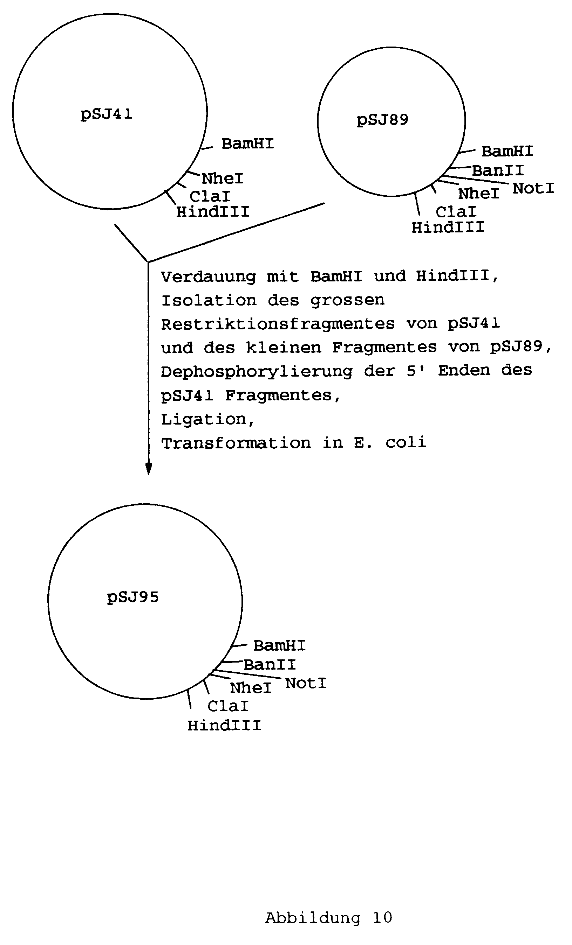

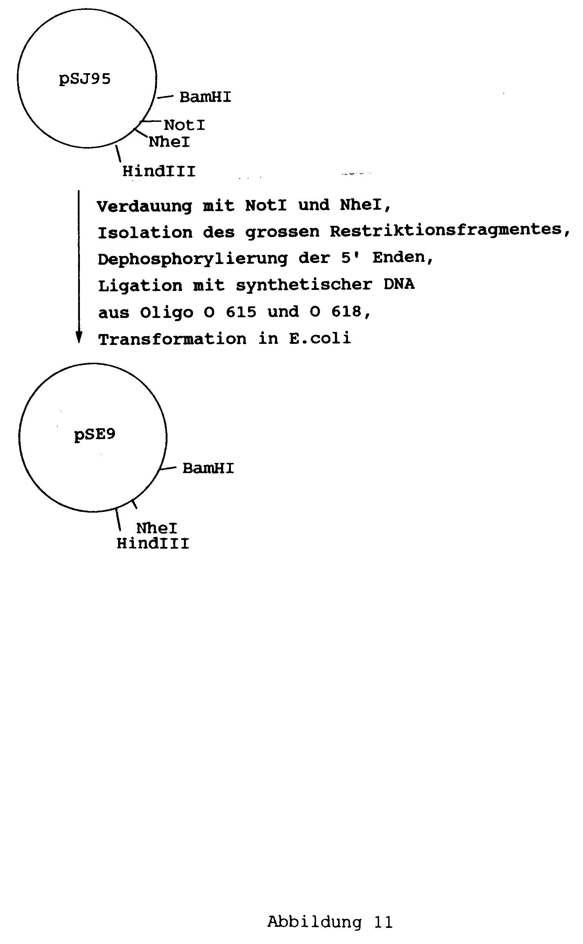

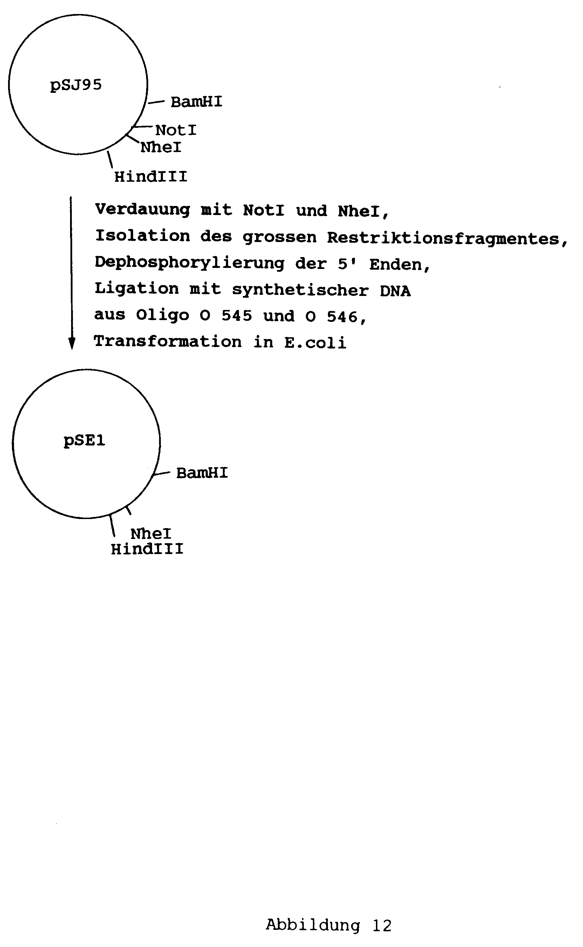

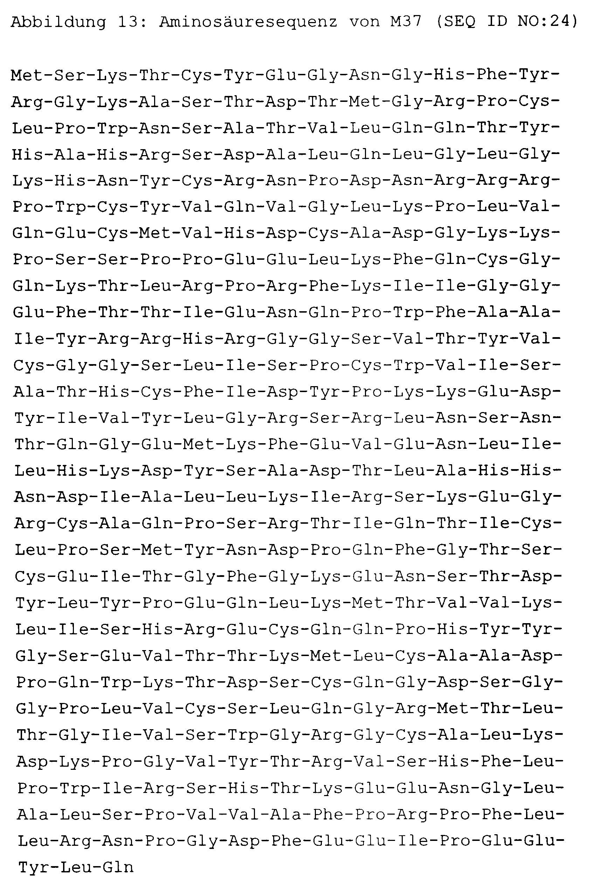

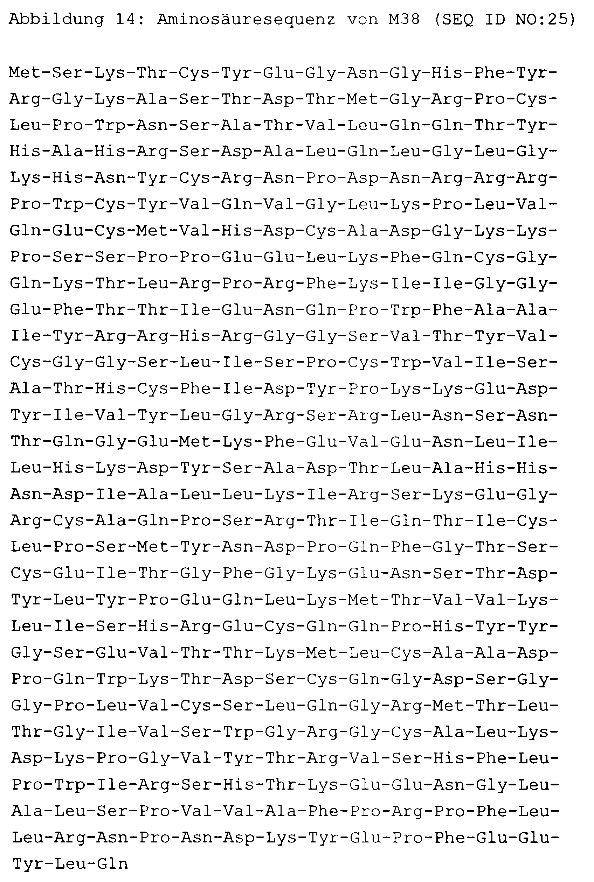

**a) Klonierungsarbeiten**
   Die Expressionsplasmide für die gentechnische Herstellung der erfindungsgemäßen Polypeptide in Escherichia coli wurden in an sich bekannter Weise hergestellt. Die Abfolge der einzelnen Herstellungsschritte ist in den Abbildungen 1 bis 12 dargestellt. Ausgangsprodukte der Plasmidherstellung waren die Plasmide pBlueskript KS II + (Firma Stratagene, Heidelberg), pUC8 (Fa. Pharmacia, Freiburg) sowie pGR201. pGR201 ist identisch mit dem in EP 408 945 und Appl. Microbiol. Biotechn. 36, 640 - 649 (1992) beschriebenen Plasmid pBF160. Die Restriktionsendonukleasen BanII, BamHI, ClaI, HindIII, NcoI, NdeI, NheI und NotI sowie die DNA-modifizierenden Enzyme, wie die alkalische Phosphatase, T4-Ligase, T4-Kinase und T7-Polymerase, wurden von den Firmen Pharmacia, Stratagene, Boehringer Mannheim und Gibco (Eggenstein) bezogen. Die Veränderungen der Plasmide während ihrer Herstellung wurden durch Restriktionsanalyse und DNA-Sequenzierung überprüft. Die DNA-Sequenzierung wurde nach den Vorschriften des Herstellers mit einer Reagenziensammlung der Firma Pharmacia durchgeführt. Bei der Herstellung der Plasmide wurden verschiedene Oligodesoxyribonukleotide (Oligos) eingesetzt, deren Sequenzen zusammen mit den zugehörigen Bezeichnungen in Tabelle 1 angegeben sind.
   Die Oligodesoxybonukleotide wurden in detrithylierter Form im 0,1 µMol-Maßstab mit einem Synthesizer (Modell 391) der Firma Applied Biosystems (Weiterstadt) nach den Angaben des Herstellers unter Verwendung von β-cyanoethyl-geschützten Diisopropylaminophosphoamiditen gefertigt. Je 100 pmol Oligodesoxyribonukleotid wurden in 50 mM Tri(hydroxymethyl)aminomethan/HCl (Tris/HCl), 10 mM Magnesiumchlorid und 5 mM Dithiothreitol bei einem pH-Wert von 7,5 mit einer Enzymeinheit T4-Kinase in Anwesenheit von 10 mM Adenosintriphosphat phosphoryliert und anschließend im gleichen Puffer zu doppelsträngigen DNA-Molekülen umgeformt. Die erhaltenen synthetischen, doppelsträngigen DNA-Moleküle wurden durch Gelelektrophorese auf einem Polyacrylamidgel (5% Polyacrylamid) gereinigt und anschließend in die Ligation mit den entsprechend vorbereiteten Plasmiden eingesetzt. Die Vorbereitung der Plasmide durch Verdauung mit Restriktionsenzymen, Isolierung der entsprechenden Restriktionsfragmente und Dephosphorylierung der 5'-Enden, die nachfolgende Ligation und die Transformation in E.coli K12 JM103 sowie alle weiteren gentechnischen Arbeiten wurden in an sich bekannter Weise durchgeführt und sind in Sambrook et al. "Molecular Cloning: A Laboratory Manual", 2. Auflage, Cold Spring Harbor Laboratory Press, Cold Spring Harbour, USA, 1989 angegeben. 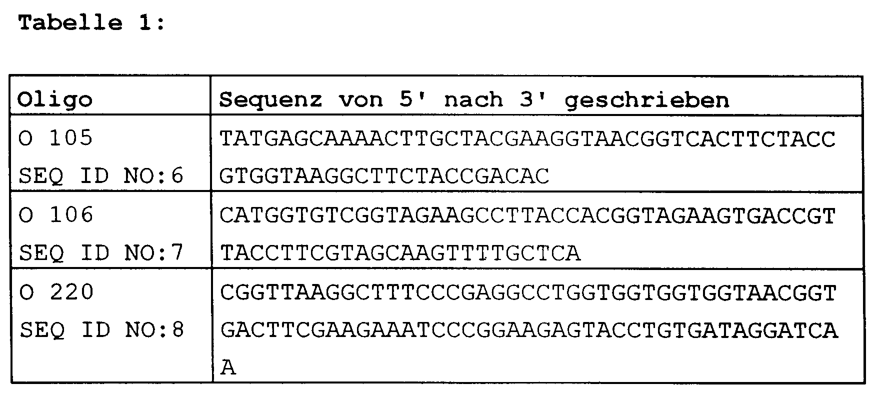 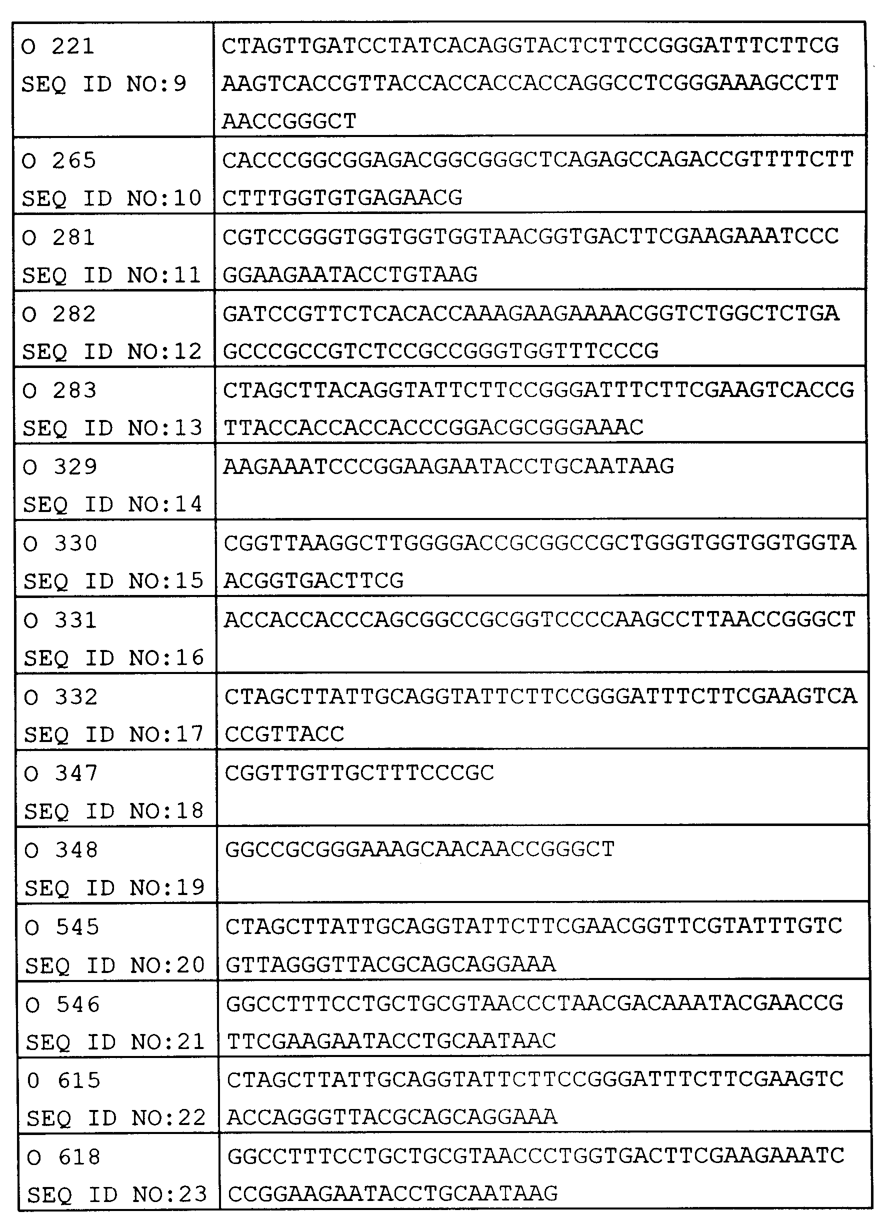
**b) Herstellung von Dauerkulturen und Fermentation**
   Die rekombinanten Expressionsplasmide pSE1 (M 38) und pSE9 (M 37) wurden in E.coli K12 JM103 (ATCC 39403) eingebracht und auf Standard-I-Nähragar (Firma Merck, 150 mg/l Ampicillin) ausgestrichen (Sambrook et al. "Molecular Cloning: A Laboratory Manual"). Jeweils eine einzelne Kolonie einer jeden Transformation wurde in Standard-I-Nährbouillon (Fa. Merck, pH 7,0; 150 mg/l Ampicillin) bei 20°C bis zur optischen Dichte (OD) von 1 bei 578 nm kultiviert, in Portionen von 2 ml als Dauerkultur unter Zusatz von Dimethylsulfoxid (DMSO) (7,5 % Endkonzentration) bei -70°C eingefroren und aufbewahrt. Zur Gewinnung der erfindungsgemäßen Polypeptide wurde jeweils 1 ml einer jeden Dauerkultur in 20 ml Standard-I-Nährbouillon (pH 7,0; 150 mg/l Ampicillin) suspendiert und bei 37°C bis zur OD von 1 bei 578 nm kultiviert.
   Anschließend wurde die gesamte Menge der erhaltenen Kultur in 1 l Standard-I-Nährbouillon (pH 7,0; 150 mg/ l Ampicillin) suspendiert und in Schüttelkolben bei 37°C fermentiert. Die Induktion erfolgte durch Zusatz von 2 ml Indolacrylessigsäurelösung (60 mg in 2 ml Ethanol) bei einer OD von 0,5 bis 1 bei 578 nm.
**c) Expressionstestung**
   Zur Testung der Expressionsrate wurden unmittelbar vor der Induktion und jede Stunde nach der Induktion (insgesamt 6 Stunden) Zellen entsprechend 1 ml einer Zellsuspension mit einer OD von 1 bei 578 nm zentrifugiert. Die sedimentierten Zellen wurden mit Lysozym (1 mg Lysozym pro ml in 50 mM Tris/HCl-Puffer, pH 8,0, 50 mM Ethylendiamintetraessigsäure (EDTA) und 15 % Saccharose) aufgeschlossen. Das Homogenat der lysierten Zellen wurde in 4 - 5 M Guanidiniumhydrochloridlösung solubilisiert und nach Verdünnen auf 1,2 M Guanidiniumhydrochlorid unter Zusatz eines Reduktionsmittels (Glutathion oder Cystein) 2 - 5 Stunden der Rückfaltungsreaktion unterworfen (Winkler et al., Biochemistry 25 4041 bis 4045 (1986)). Die erhaltenen einkettigen erfindungsgemäßen Polypeptide wurden durch Zugabe von Plasmin in die entsprechenden zweikettigen Moleküle umgewandelt, deren Aktivität mit dem chromogenen Substrat pyro-Glu-Gly-Arg-p-nitroanilid bestimmt wurde. Die Aktivierung der erfindungsgemäßen Polypeptide mit Plasmin erfolgte in 50 mM Tris/HCl-Puffer, 12 mM Natriumchlorid, 0,02 % Tween 80 bei pH 7,4 und 37°C. Das Verhältnis erfindungsgemäßes Polypeptid zu Plasmin lag bei etwa 8.000 - 36.000 zu 1, bezogen auf Enzymeinheiten. Die Testinkubation erfolgte in 50 mM Tris/HCl-Puffer und 38 mM Natriumchlorid bei pH 8,8 in Gegenwart von 0,36 µM Aprotinin (zur Hemmung des Plasmins) und 0,27 mM Substrat pyro-Glu-Gly-Arg-p-nitroanilid bei 37°C. In Abhängigkeit von der Konzentration an erfindungsgemäßem Polypeptid wurde die Reaktion nach 5 bis 60-minütiger Inkubation durch Zusatz von 50 %-iger Essigsäure gestoppt und die Extinktion bei 405 nm gemessen. Gemäß den Angaben des Herstellers des Substrats (Kabi Vitrum, Schweden) entspricht bei dieser Vorgehensweise eine Extinktionsänderung von 0,05 pro Minute bei 405 nm einer Urokinase-Aktivität von 25 Ploug-Einheiten pro ml Testlösung. Die erfindungsgemäßen Polypeptide wiesen spezifische Aktivitäten zwischen 120.000 und 155.000 Ploug-Einheiten pro mg Protein auf. Der Proteingehalt der Lösungen wurde mit dem BCA-Assay der Firma Pierce bestimmt.
**d) Isolierung und Reinigung**
   Nach 6 Stunden wurde die unter den in 1b) beschriebenen Bedingungen durchgeführte Fermentation beendet (Dichte 5 - 6 OD bei 578 nm), und die Zellen wurden durch Zentrifugation gewonnen. Das Zellsediment wurde in 200 ml Wasser resuspendiert und im Hochdruckhomogenisator aufgeschlossen. Nach erneuter Zentrifugation wurde der Niederschlag, der die gesamte Menge an einkettigem, erfindungsgemäßem Polypeptid enthielt, in 500 ml 5 M Guanidiniumhydrochlorid, 40 mM Cystein, 1 mM EDTA bei einem pH-Wert von 8,0 gelöst und mit 2000 ml 25 mM Tris/HCl mit einem pH-Wert von 9,0 verdünnt. Die Rückfaltungsreaktion war nach ca. 12 Stunden abgeschlossen.
   Die erhaltenen erfindungsgemäßen Polypeptide wurden nach Zusatz von 8 g Kieselgel durch 2-stündiges Rühren vollständig an Kieselgel gebunden. Das beladene Kieselgel wurde abgetrennt und mit Acetat-Puffer (pH 4,0) gewaschen. Die Polypeptide wurden mit 0,5 M Trimethylammoniumchlorid (TMAC) in 0,1 M Acetat-Puffer (pH 4) eluiert. Nach zwei chromatographischen Trennungen (Kupfer-Chelat-Säule und Kationenaustauscher) wurden die Polypeptide in reiner Form erhalten. Durch N-terminale Sequenzanalyse wurde die Einkettigkeit nachgewiesen.
   Die isolierten erfindungsgemäßen Polypeptide, deren Aminosäuresequenzen in den Abbildungen 13 bis 14 angegeben sind, zeigten in einem direkten Aktivitätstest mit dem chromogenen Substrat für Urokinase keine oder nur sehr geringe Aktivität (unter 1 %). Erst nach Spaltung mit Plasmin (Bedingungen sind in Abschnitt 1c) angegeben) wurde die volle Enzymaktivität erhalten. Die erfindungsgemäßen Polypeptide wurden demnach in E.coli K12 JM103 als einkettige Proteine exprimiert.

### 2. Bestimmung der thrombinhemmenden Wirkung

Die Inhibitoraktivität der erfindungsgemäßen Polypeptide wurde durch Messung der Thrombinzeit bestimmt, indem 200 µl einer 1 : 10 Verdünnung an humanem Citratplasma in Veronalpuffer mit 50 µl Thrombinlösung (0,2 Einheiten) und 50 µl einer wäßrigen Lösung enthaltend 0,4 - 30 µg eines erfindungsgemäßen Polypeptides gemischt wurden. Dann wurde die Zeit bis zur Bildung eines Fibringespinnst gemessen (Thrombinzeit).

Die in Tabelle 2 aufgeführten Thrombinzeitwerte wurden in Anwesenheit von Prourokinase oder der erfindungsgemäßen Proteins M 37 und M 38 bestimmt. Im Gegensatz zu Prourokinase verlängern M 37 und M 38 dosisabhängig die Thrombinzeit und wirken somit als Inhibitoren der Gerinnung.

**Tabelle 2**

| Protein [µg] | Thrombinzeit [sec] | | |
|---|---|---|---|
| | Prourokinase | M37 | M38 |
| 0 | 31 | 32 | 32 |
| 0,4 | | 40 | |
| 0,8 | | 79 | |
| 1,2 | | 148 | |
| 1,6 | | 195 | |
| 2,0 | | 266 | |
| 4,0 | 31 | >300 | 58 |
| 8,0 | | | 81 |
| 12,0 | | | 104 |
| 16,0 | | | 130 |
| 20,0 | 33 | | 150 |
| 30,0 | 33 | | >300 |

## Patentansprüche

1. Chimäre Proteine mit fibrinolytischen und thrombinhemmenden Eigenschaften, die am C-terminalen Ende der Plasminogen aktivierenden Aminosäuresequenz mit einer Aminosäuresequenz der Formel I (SEQ ID NO:1)
Ser-X₁-X₂-X₃-X₄-X₅-Pro-Arg-Pro-Y₁-Y₂-Y₃-Y₄-Asn-Pro-Z
verknüpft sind, in der X₁ Pro oder Leu, X₂ Gly, Val oder Pro, X₃ Lys, Val, Arg, Gly oder Glu, X₄ Ala, Val, Gly, Leu oder Ile, X₅ Gly, Phe, Trp, Tyr oder Val, Y₁ Phe, Tyr oder Trp, Y₂ Leu, Ala, Gly, Ile, Ser oder Met, Y₃ Leu, Ala, Gly, Ile, Ser oder Met, Y₄ Arg, Lys oder His und Z die Aminosäuresequenz der Formel II (SEQ ID NO:2)
Gly-Asp-Z₁-Glu-Glu-Ile-Pro-Glu-Glu-Tyr-Leu-Gln
mit Z₁ Phe oder Tyr
oder der Formel III (SEQ ID NO:3)
Asn-Asp-Lys-Tyr-Glu-Pro-Phe-Glu-Glu-Tyr-Leu-Gln
oder der Formel IV (SEQ ID NO:4)
Ser-Asp-Phe-Glu-Glu-Phe-Ser-Leu-Asp-Asp-Ile-Glu-Gln
oder der Formel V (SEQ ID NO:5)
Ser-Glu-Phe-Glu-Glu-Phe-Glu-Ile-Asp-Glu-Glu-Glu-Lys
bedeuten.

2. Proteine nach Anspruch 1, dadurch gekennzeichnet, daß die Plasminogen aktivierende Aminosäuresequenz die unveränderte Aminosäuresequenz der Prourokinase, mindestens eine durch Deletion, Substitution, Insertion und/oder Addition modifizierte Aminosäuresequenz der Prourokinase, die unveränderte Aminosäuresequenz der Urokinase, mindestens eine durch Deletion, Substitution, Insertion und/oder Addition modifizierte Aminosäuresequenz der Urokinase, die unveränderte Aminosäuresequenz des Gewebeplasminogenaktivators (t-PA), mindestens eine durch Deletion, Substitution, Insertion und/oder Addition modifizierte Aminosäuresequenz von t-PA, die unveränderte Aminosäuresequenz des Fledermaus-Plasminogenaktivators (bat-PA), mindestens eine durch Deletion, Substitution, Insertion und/oder Addition modifizierte Aminosäuresequenz von bat-PA und/oder die Aminosäuresequenz von Streptokinase, Staphylokinase und/oder APSAC enthält.

3. Proteine nach Anspruch 2, dadurch gekennzeichnet, daß die Plasminogen aktivierende Aminosäuresequenz die unveränderte Aminosäuresequenz von Prourokinase, mindestens eine durch Deletion, Substitution, Insertion und/oder Addition modifizierte Aminosäuresequenz der Prourokinase, die unveränderte Aminosäuresequenz von t-PA und/oder mindestens eine durch Deletion, Substitution, Insertion und/oder Addition modifizierte Aminosäuresequenz von t-PA enthält.

4. Proteine nach Anspruch 3, dadurch gekennzeichnet, daß die Plasminogen aktivierende Aminosäuresequenz aus der aus 411 Aminosäuren bestehenden Sequenz der Prourokinase, in der die Aminosäure in Position 407 Asn oder Gln ist, aus der Aminosäuresequenz ⁴⁷Ser bis ⁴¹¹Leu der Prourokinase, in der die Aminosäure in Position 407 Asn oder Gln ist, aus der Aminosäuresequenz ¹³⁸Ser bis ⁴¹¹Leu der Prourokinase, in der die Aminosäure in Position 407 Asn oder Gln ist, aus der unveränderten, aus 527 Aminosäuren bestehenden Sequenz von t-PA, aus der Aminosäuresequenz Ser-⁸⁹Arg bis ⁵²⁷Pro von t-PA und/oder aus der Aminosäuresequenz ¹⁷⁴Ser bis ⁵²⁷Pro von t-PA besteht.

5. Proteine nach einem oder mehreren der Ansprüche 1 bis 4, dadurch gekennzeichnet, daß in der Aminosäuresequenz der Formel I X₁ Pro, X₂ Val, X₃ Lys oder Val, X₄ Ala und X₅ Phe bedeuten.

6. Proteine nach einem oder mehreren der Ansprüche 1 bis 5, dadurch gekennzeichnet, daß in der Aminosäuresequenz der allgemeinen Formel I Y₁ Phe, Y₂ Leu, Y₃ Leu und Y₄ Arg bedeuten.

7. Proteine nach einem oder mehreren der Ansprüche 1 bis 6, dadurch gekennzeichnet, daß in der Aminosäuresequenz der Formel I Z die Aminosäuresequenz der Formel II oder der Formel IV bedeutet.

8. Plasmide zur Gewinnung eines chimären Proteins gemäß den Ansprüchen 1 bis 7, dadurch gekennzeichnet, daß das Operon einen regulierbaren Promotor, eine als Ribosomenbindestelle wirksame Shine-Dalgarno-Sequenz, ein Startcodon, ein synthetisches Strukturgen für ein chimäres Protein gemäß den Ansprüchen 1 bis 7 und stromabwärts vom Strukturgen 1 oder 2 Terminatoren aufweist und daß die Plasmide zur Expression eines chirmären Proteins mit fibrinolytischen Eigenschaften in Stämmen von Escherischia coli geeignet sind.

9. Plasmide nach Anspruch 8, ausgewählt aus der Gruppe pSE1 und pSE9.

10. Verfahren zur Herstellung von Plasmiden nach einem oder beiden der Ansprüche 8 bis 9, dadurch gekennzeichnet, daß man diese aus den Plasmiden pBlueskript KS II+, pUC8 und pGR201 gemäß Abbildungen 1 bis 12 gewinnt.

11. Verwendung eines Plasmids nach einem oder beiden der Ansprüche 8 bis 9 zur Gewinnung eines chimären Proteins gemäß Ansprüchen 1 bis 7, dadurch gekennzeichnet, daß man mit einem Plasmid einen Escherischia coli-Stamm in an sich bekannter Weise transformiert, die Expression des Strukturgens induziert, das gebildete Vorstufenprotein vom Medium und den lysierten Bakterienzellen abtrennt, solubilisiert und anschließend durch Einwirkung eines Redox-Systems zum Protein mit fibrinolytischen Eigenschaften rückfaltet.

12. Thrombolytikum, das als Wirkstoff ein chimäres Protein gemäß den Ansprüchen 1 bis 7 enthält.
